# EUROPEAN PATENT SPECIFICATION

(11) **EP 0 858 502 B1**
(45) Date of publication and mention of the grant of the patent: **16.02.2005**
(21) Application number: 96939464.2
(22) Date of filing: 15.10.1996
(51) Int. Cl.: C12N 5/06, C12N 5/08, A61K 35/30

(54) **NORMAL NEURAL EPITHELIAL PRECURSOR CELLS**
VORLÄUFER VON NORMALEN NEURALEN EPITHELZELLEN
CELLULES PRECURSEURS EPITHELIALES NEURALES NORMALES

(30) Priority: 01.11.1995 US 548345
(43) Date of publication of application: 19.08.1998
(73) Proprietor: Genentech, Inc., South San Francisco, CA 94080-4990 (US)
(72) Inventor: LI, Ronghao, La Jolla, California 92037 (US); MATHER, Jennie, Millbrae, CA 94030 (US)
(74) Representative: Walton, Seán Malcolm
(86) International application number: PCT/US1996/016543
(87) International publication number: WO 1997/016534

(56) References cited:
- WO-A-94/02593
- WO-A-96/35776
- ENDOCRINE, vol. 5, no. 2, October 1996, NEW YORK, N.Y., US, pages 205-217, XP000647380 R. LI ET AL.: "MULTIPLE FACTORS CONTROL THE PROLIFERTATION AND DIFFERENTIATION OF RAT EARLY EMBRYONIC (DAY 9) NEUROEPITHELIAL CELLS."

## Description

### Field of the Invention

The present invention relates to methods for isolating normal non-human neural epithelial precursor cells that are capable of cell replication, expansion, and long term culture in vitro while maintaining their ability for differentiation in vitro and in vivo. The methods for isolating the normal neural epithelial precursor cell type of the present invention result in a substantially homogeneous population of non-transformed, non-tumorigenic neural epithelial precursor cells. Therefore, the invention also relates to normal neural epithelial precursor cell lines of non-human mammalian origin. The isolated neural epithelial precursor cells of the invention can be maintained and expanded in culture employing the methods described herein.

### Description of Related Disclosures

The mammalian central nervous system is developmentally derived from neuroepithelial cells in the neural tube (Murphy, M. et al., J. Neurosci. Res. (1990) 25:463-475; see generally,"Developmental Biology" Fourth Edition, Gilbert, S., (1994) Sinauer Associates, Inc.; "Post Implantation Mammalian Embryos: A Practical Approach", Copp, AJ., and Cockroft, D.L., eds. (1990) Oxford University Press, New York). In vivo, neuroepithelial cells grow and differentiate to different types of neuronal and glial cells in response to, as yet unidentified, environmental factors. The ability to isolate and maintain particular neural precursor cells in vitro is crucial to the identification of these factors. Additionally, the ability to isolate and expand non-tumorigenic neural cell types at early embryonic stages of development is critical to the success ofcell based therapies including intracerebral grafting and gene transfer (Gage et al., (1991), TINS, 14:8328-333; Mucke and Rockenstein, (1993) Transgene, 1:3-9; Jiao et al., (1992) Brain Res. 575:143-147).

Broadly speaking, there are two types of cell culture for the maintenance and characterization of cells of the mammalian nervous system (Bottenstein, J.E., "Growth and Differentiation of Neural Cells in Defined Media," Cell Culture in the Neurosciences, Bottenstein and Sato, eds., pp 3-43 [1985]). Primary culture involves tissue explant and subsequent culture of either the dispersed cells or the explanted tissue for the finite lifetime of the dispersed cells or tissue. After several days in culture the cells generally spontaneously differentiate into a variety of neuronal cell types (Cattaneo and McKay (1990) Nature 347:762-765). The primary advantage of dissociated cell culture is that single cell types can be studied as representative of actual in vivo cell types ("Developmental Biology of Cultured Nerve, Muscle, and Glia," David Shubert, John Wiley & Sons, Inc. (1984)). Using primary culture heterogenous samples of cells can be isolated to achieve representative cell populations of uniform phenotype. Many populations of cells derived from explanted tissue have, heretofore, been limited in the number of times they can divide possibly owing to in vitro aging (Orgel, L.E. (1973) Nature, 243:441-445) or inappropriate culture conditions.

Continuous cell culture represents a second variety of in vitro cell culture for cells of the developing mammalian central nervous system (Bottenstein, J. (1985) supra). Continuous cell culture provides the advantage of long term culture and storage. Since the proliferation and differentiation of neuroepithelial cells is a programmed process in vivo and neuroepithelial cells cultured in vitro tend to differentiate spontaneously after limited divisions, long term culture of neuroepithelial cells has previously relied on viral or oncogene transformation.

Several groups have reportedly established multipotent neural cell lines through retroviral gene transfer (Snyder, et al.. Cell, (1992) 68:33-51; Ryder et al., J. Neurobiol, (1990) 21:356-375; Geller and Dubois-Dalq, (1988) J. Cell Biol. 107:1977-1986; Bartlett, (1988) Proc. Natl. Aced. Sci. USA 85:3255-3259; Birren and Anderson (1990) Neuron 4:189.201; Evrad, et al., (1990) Proc. Natl. Acad. Sci. USA 87:3062-3066; and Frederikson. et al. (1988) Neuron, 1:439-448). Oncogenes generally used to establish cells from the developing nervous system include *v-myc,* SV40 T antigen, and *neu* (Frederiksen et al., (1988) Neuron, 1:439-448; Gao and Hatten (1994)Development 120:1059-1070). The cell lines can be identified by genotypic alterations including the expression of exogenous genes such as *lacZ* (Snyder et al., (1992) supra). Transformed cell lines can be established from embryonic cells at various stages of development. Snyder et al. established their v-*myc*-immortalized cells from embryonic day 13 external germinal layer derived cerebellar cells (Snyder et al.. (1992) supra).

Others have used embryonal carcinoma (EC) cells to study neural differentiation since EC cells can be obtained in large amounts and can differentiate into cells of various phenotypes (Edde, B. and Darmon M., "Neural Differentiation of Pluripotent Embryonal Carcinoma Cells." Cell Culture in the Neurosciences, Bottenstein and Sato. eds., 273-285 [1985]).

Viral transfection and oncogene transformation can halt the cell differentiation program at specific stages of differentiation (Frederiksen, et al., (1988) Neuron 1:439-448). These methods also result in alteration of the neural precursor cell properties. SV40 large T-antigen oncogene subverted the establishment of granule cell identity (Gao and Hanen. (1994) Development 120:1059-1070). Because of their transformed phenotype and their potential for invasive growth in vivo, these cells are unlikely candidates for cell based therapies (Jiao et al. (1992) Brain Res. 143-147).

Recently. evidence suggests that it should be possible to halt neuroepithelial cell differentiation and allow for infinite proliferation of untransformed murine neural precursor cells in serum free medium supplemented with appropriate growth factors (Loo et al., (1987) Science 236:200-202: Loo et al., (1991) J. Neurosci. Res. 28:101-109). Unlike mouse embryo cells cultured in conventional serum supplemented media these cells do not lose their proliferative potential leading to genetically altered cell lines (Loo et al., (1987) Science. 236:200-202). Researchers have established cell lines from 16 day old embryos which display an absolute requirement for epidermal derived growth factor (EGF) (Loo et al., (1989) J. Cell. Physiol. 139:484-491). An immortal glial precursor cell line (SFME) has been established in the presence of EGF (Loo et al., (1987) supra). Cells have also been isolated from embryonic day 10 mice which have an absolute requirement for fibroblast growth factor (FGF) (Murphy, et al., (1994) J. Neurosci. Res. 25:463-475).

Serum free embryo cells similar to the mouse SFME cells can be derived from embryonic human brain at similar stages of development using EGF (Loo et al., (1991) J. Neurosci. Res. 28:101-109). However, these cell lines, derived from 20-24 week old human embryos ceased proliferation after about 70 population doublings (Loo et al. (1991) supra). EGF responsive cells have also been identified in adult mammalian central nervous system (Reynolds and Weiss Science (1992) 225:1707-1710). EGF was found to support the indefinite growth of subvetricular neural progenitor cells from adult mouse and retinal neural precursor cells (Reynolds and Weis (1992) supra).

It has been demonstrated that basic fibroblast growth factor (bFGF) is a potent mitogen for certain CNS populations (Murphy et al., (1990) supra; Cattaneo and McKay (1990) supra; Ghosh and Greenberg (1995) Neuron 15:89-103; and Vicario-Abejon et al. (1995) Neuron 15:105-114). It also appears that bFGF and nerve growth factor (NGF) cooperatively promote proliferation and differentiation of embryonic striatal neurons (Cattaneo and McKay (1990) supra) However, neither EGF nor FGF support neuroepithelial cells cultured from earlier stages of development as, for example, embryonic day 9 rat neural tube.

Good cellular candidates for cell based therapies such as intracerebral grafting and gene transfer are optimally free of genetic modification and readily available. Additionally, good candidates will exhibit prolonged in vivo survival and non-invasive growth (Gage et al., (1991) TINS 14:328-333): Recently, immortalized neural precursor cells have been used as a carrier for the local delivery of NGF in the brain (Martinez-Serrono (1995) Neuron 15:473-484). The NGF secreting cells expressed a transgene and secreted bioactive nerve growth factor at levels sufficient to reverse cholinergic neuron atrophy in transplanted rats (Martinez-Serrono et al., (1995) supra). These cells were able to survive up to 10 weeks after transplantation and reverse age-dependent cognitive impairments in the model. Another group has transplanted human fetal mesencephalic tissue into the caudate nucleus of patients with Parkinson's disease (Spencer et al. (1992) New England J. Med. 327:1541-1548). Intracerebral grafting has been demonstrated with non-neuronal cell types such as fibroblasts (Gage et al. (1991) Trends Neurosci. 14:328-333; Gage and Fisher, et al., (1991) Neuron 6:1-12; Fisher (1994) Neurochem. Int, 25:47-52) and primary muscle cells (Jiao et al., (1992) 575:143-147) in part to avoid the possibility oftumorigenesis and helper virus contamination that may impact immortalized primary neuronal cells in vivo.

Others have used genetically altered polymer-encapsulated cells to deliver human nerve growth factor transgene product in rat models (Winn et al., (1994) Proc. Natl. Acad. Sci. 91:2324-2328; Hoffman, et al., (1993) Experimental Neurology 122:100-106;Maysinger et al., (1994) Neurochem. Int. 24:495-503). Polymer encapsulation allows diffusion of macromolecules and permits nutrient exposure (Winn et al., (1994) supra).

Snyder et al. have shown that early embryonic precursor cells can engraft and participate in the development of mouse cerebellum (Snyder et al., (1992) Cell 68:33-51)

There is a need therefore for normal neural epithelial cells from early embryonic stage of development that are capable of replication and expansion both in vitro and in vivo for a variety of purposes including cell based therapies.

### Summary of the Invention

It has now been discovered that a neural precursor cell type can be isolated from non-human mammalian embryonic neural tissue that is capable of continuous in vitro cell culture. The cell type, which is isolated in serum free culture from non-human embryonic neural tissue, does not respond to epidermal growth factor, does not display phenotypic aberration by virtue of viral transfection or oncogene transformation, and can be maintained in continuous culture without undergoing a growth crisis, senescence, or spontaneous differentiation. This cell type has been identified as a neural epithelial precursor cell (NEP cell) expressing the intermediate filament protein nestin. The present invention therefore provides a method for isolating a normal neural epithelial precursor cell type from a developing non-human mammalian embryo.

According to the present invention when neural tissue from the developing non-human mammalian central nervous system at the zero-somite stage of development, equivalent to the rat embryonic day 9, is cultured in the presence of embryonic Schwann cells or embryonic Schwann cell conditioned media, a neural epithelial precursor cell type can be isolated that can be maintained in continuous culture in serum free media. Therefore, the present invention provides a method of isolating a normal neural epithelial precursor cell type comprising the steps of culturing neural tissue from a non-human mammalian embryo at the zero-somite stage of development in the presence of embryonic Schwann cells or embryonic Schwann cell conditioned media and isolating the normal neural epithelial precursor cells.

The invention further provides for the normal neural epithelial cell type isolated according to the methods of the present invention. The neural epithelial cell line of the present invention is of non-human mammalian origin. According to one aspect of the present invention neural tube from a rat embryo at the ninth day of development is cultured according to the methods of the present invention. According to this aspect of the present invention a neural epithelial precursor cell line of rat origin is obtained.

The invention further provides a method for the long term culture, expansion, and maintenance of the cell lines of the present invention. According to the methods of the present invention, the normal neural epithelial precursor cell type can be maintained in long term culture. Preferably the cells are cultured on plates coated with laminin, or extracellular matrix materials containing laminin.

The invention further relates to compositions comprising the normal neural epithelial precursor cells of the present invention. The compositions of the present invention are formulated in a pharmaceutically acceptable vehicle. In one embodiment the phamaceutically acceptable vehicle is a balanced saline solution. In a further embodiment a solid or semisolid, gelatinous, or viscous support medium is the pharmaceutically acceptable vehicle. Preferred gelatinous vehicles include collagen and hydrogels optionally supplemented with . the relevant extracellular matrix proteins such as laminin and may include the appropriate growth factors. Such compositions are suitable for transplantation into either the peripheral or central nervous system.

### Brief Description of the Drawings

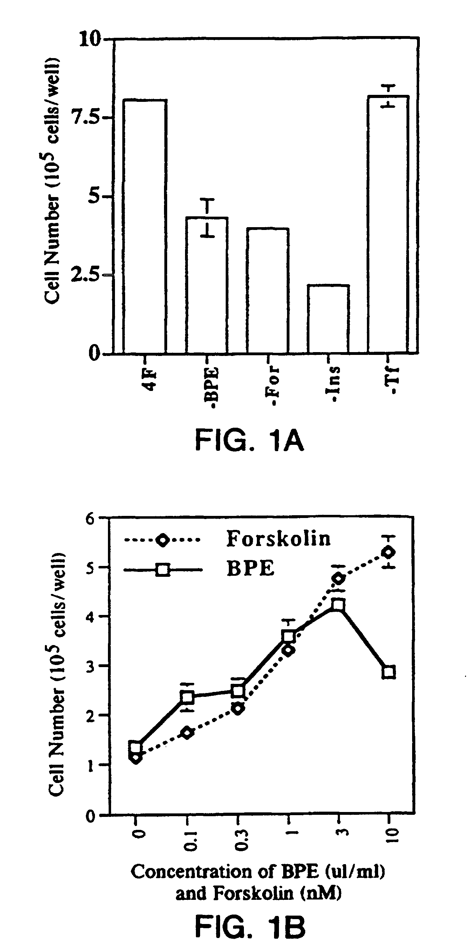

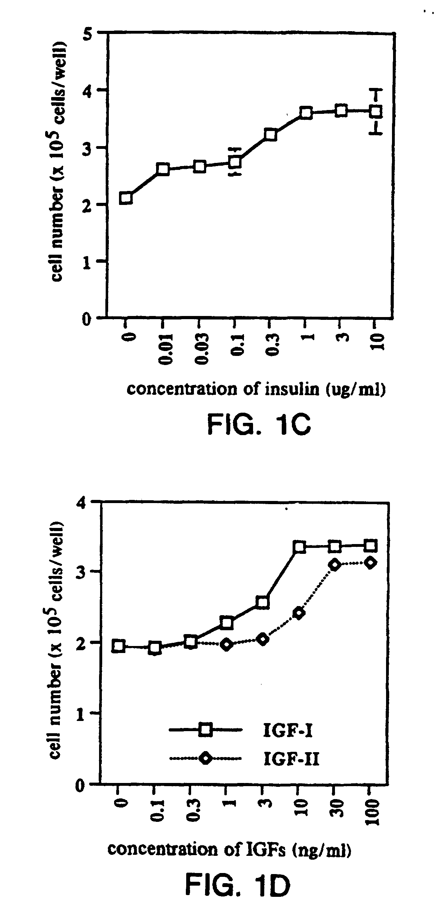

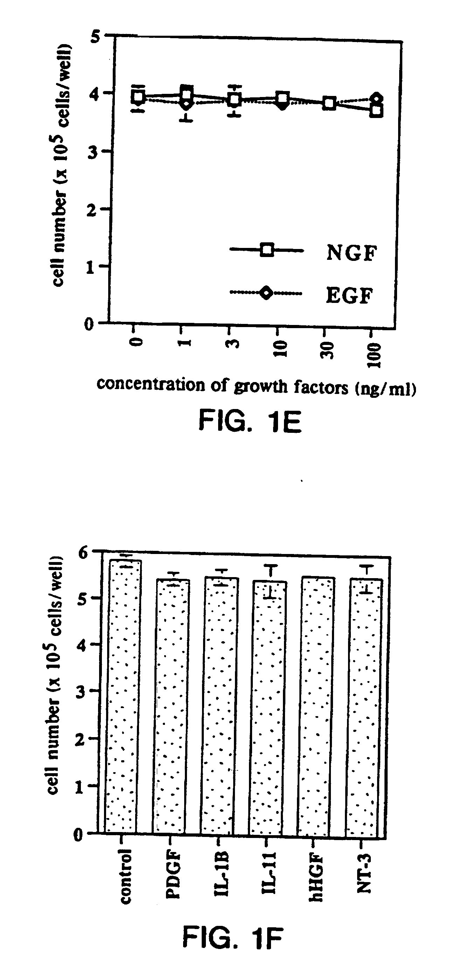

Figure 1A-Figure 1F. Neural epithelial precursor (NEP) cells isolated according to the methods described herein were cultured in the presence and absence of various factors. For Figure 1A cells were grown in F12/DME supplemented with bovine pituitary extract(2 µg/ml), forskolin (5 µM), insulin (10 µg/ml) and transferrin (10 µg/ml) (lane 4F) or in the same media excluding i) bovine pituitary extract (lane BPE), ii) forskolin (lane For), and iii) insulin (lane Ins). Figures 1B-1E describe the results of culturing NEP cells in F12/DME containing bovine pituitary extract (BPE), insulin, heregulin (1 nM), Vitamin E (5 µg/ml), progesterone (3 nM), transferrin (10 µg/ml), and forskolin in wells coated with laminin in the presence of increasing concentrations of forskolin and bovine pituitary extract (Figure 1B), insulin (Figure IC), insulin like growth factors I and II (Figure 1D), nerve growth factor and epidermal growth factor (Figure 1E). NEP cells in Figure 1F were cultured in F12/DME containing bovine pituitary extract (BPE) (2 µg/ml), insulin (10 µg/ml), heregulin (1nM), Vitamin E (5 µg/ml), progesterone (3 nM), transferrin (10 µg/ml), and forskolin (5 µM) on laminin coated plates and in the presence or absence of platelet derived growth factor (PDGF), interleukin I B (IL-1B), interleukin 11 (IL-11), human hepatocyte growth factor (hHGF) and neurotrophin 3 (NT-3).

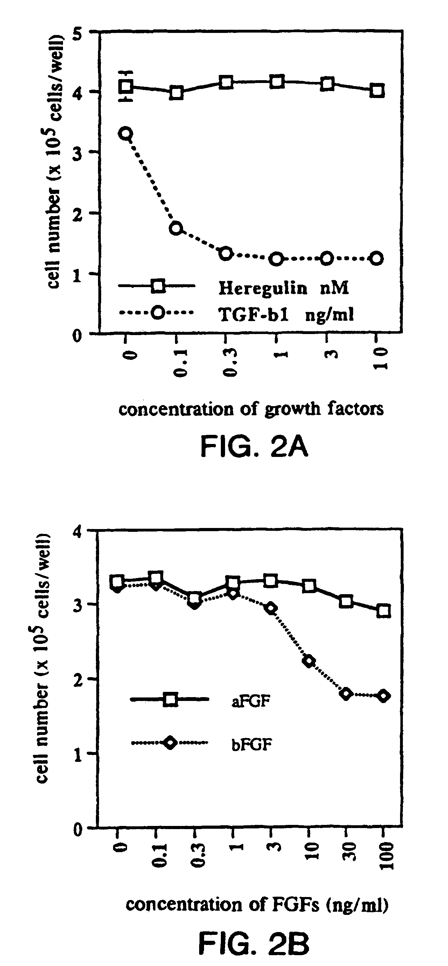

Figure 2A-Figure 2B. NEP cells were grown in DME/F12 including insulin, transferrin, bovine pituitary extract, forskolin, progesterone, and vitamin E and exposed to the indicated concentrations of heregulin or transforming growth factor ß (Figure 2A) or acidic or basic fibroblast growth factor (Figure 2B).

### Detailed Description of the Preferred Embodiments

### Definitions

The term "normal" when used in connection with a neural epithelial precursor cell type of the present invention is meant to refer to a cell type which does not display a phenotypic aberration by virtue of viral transfection or oncogene transformation. The normal cell type of the present invention can be maintained in continuous culture. are capable of replication, expansion, and long term culture in vitro while maintaining their ability for differentiation in vitro and in vivo without undergoing a growth crisis, senescence. or spontaneous differentiation. Such cell types are to be distinguished from cell lines that are capable of unlimited proliferative potential and of long term continuous culture only after infection with a transforming vehicle such as adenovirus. SV40. ras, or c-myc. Normal cell lines are non-tumorigenic. By non-tumorigenic is meant that the cell will not give rise to a tumor when injected into syngeneic or immuno-compromised animals.

A "cell line" according to the present invention, is a substantially homogeneous group or population of cells derived through the culture of a sample of cells from a tissue or organ. By "substantially homogeneous" is meant a population of cells that by virtue of common parent or parents are generally phenotypically and genotypically identical. It is to be understood that such uniform cell preparations will deviate as a result of natural mutational changes (allelic variations) that may confer variations in the phenotype in the population of cells. Such natural variations are meant to be included within the definition of substantially homogeneous and therefor are included in the definition of cell line as defined herein. Preferably, "substantially homogeneous" means that between about 90 to 100%. and preferably 99% to 100% of the cells in the population are identical cells.

"Immortalization" is the transformation of a cell culture in vitro into a strain with unlimited growth potential.

"Neural epithelial precursor (NEP) cells" are identified by the expression of the intermediate filament protein nestin (Frederickson and McKay (1988) J. Neurosci. 8:1444-1151; Lendahl, et al., (1990) Cell 60:585-595: Cattaneo and McKay (1990)Nature 347:762-765; Hockfield, S. and McKay R.D.G. (1995) J. Neurosci. 5.3310-3328). The expression ofnestin distinguishes neural epithelial precursor cells from more differentiated cells of neural tube origin (Lendahl, et al, (1990) Cell, 60:585-595; Reynolds and Weiss, (1992) Science, 225:1707-1710). The cells are isolated from neural tissue developmentally equivalent to embryonic day 9 rat neural tube. Neither EGF nor FGF are mitogenic for NEP cells, and they differentiate into a variety of neural cell types in the presence of basic fibroblast growth factor and forskolin.

By "developmentally equivalent" is meant that a particular source of embryonic tissue is obtained from a developing non-human mammalian embryo at an equivalent stage of embryonic development Embryonic development can be compared between species by the appearance of distinct morphological features. This is distinguishable from gestational time since the timing of appearance of principal morphological features varies between species. According to the present invention, non-human mammalian embryonic tissue is obtained at a stage equivalent to the embryonic zero-somite stage of development. According to the present invention a non-human mammalian embryo is obtained that is developmentally equivalent to a rat embryo at the ninth day of embryonic development. According to the present invention, rat embryonic day nine is measured by mating male and female rats for two hours between the hours of 8 and 10 a.m. and immediately seeking a copulatory plug. The day the copulatory plug is found is defined as day zero of the gestation. For other non-human mammalian species however, it is not the gestational day but the developmental stage of the embryo that is important. Therefore, according to the present invention the rat E9 embryo or the developmentally equivalent embryo from other non-human mammalian species is employed. The rat E9 embryo is generally at the zero-somite stage of development characterized by the appearance of the primitive groove and the allantois, coupled with amnion formation. For developmentally equivalent stages in other mammalian embryos the reader is directed to Kaufman, M. H., "Morphological Stages of Post Implantation Embryonic Development" in Post implantation Mammalian Embryos:A Practical Approach, Copp A., and Cockcroft, D., Eds, Oxford University Press, New York (1990), pp 81-91; Downs and Davies, (1993) 118:1255-1266; and Fujinaga and Baden (1992) Teratology 45:661-670; and O'Rahilly, R. and Müller, F. (1987), "Developmental Stages in Human Embryos," Carnegie Institution of Washington, Publication no. 637. Carnegie Institute, Washington DC.

A "Schwann cell" is a cell of neural crest origin that forms a continuous envelope around each nerve fiber of peripheral nerves in situ. A Schwann cell can be identified as such by detecting the presence of one or more Schwann cell markers such as glial fibrillar acidic protein (GFAP), or S100 protein, *e*.*g*., using antibodies against these markers. Furthermore, Schwann cells have a characteristic morphology which can be detected by microscopic examination of cultures thereof. Isolated Schwann cells can also be evaluated for the maintenance of differentiated Schwann cell functions, such as the ability to associate with sensory neurons in culture or the ability to produce myelin or myelin related proteins such as Po and myelin associated glycoprotein (MAG). An "embryonic Schwann cell" is a Schwann cell or Schwann cell precursor isolated from an embryonic non-human mammal at a stage developmentally equivalent to the rat embryonic day 14 up to birth. Preferably, the embryonic Schwann cell is isolated from a non-human mammal at a stage developmentally equivalent to rat embryonic day 14 through rat embryonic day 18, and most preferably at about embryonic day 15.

The terms "cell culture medium" and "culture medium" refer to a nutrient solution used for growing mammalian cells that typically provides at least one component from one or more of the following categories:
1) an energy source, usually in the form of a carbohydrate such as glucose;
2) all essential amino acids, and usually the basic set of twenty amino acids plus cystine;
3) vitamins and/or other organic compounds required at low concentrations;
4) free fatty acids; and
5) trace elements, where trace elements are defined as inorganic compounds or naturally occurring elements that are typically required at very low concentrations, usually in the micromolar range.

The nutrient solution may optionally be supplemented as described herein with one or more components from any of the following categories:
1) one or more mitogenic agents:
2) salts and buffers such as, for example, calcium. magnesium, and phosphate:
3) nucleosides and bases such as, for example, adenosine and thymidine. hypoxanthine: and
4) protein and tissue hydrolysates.

According to the present invention the cell culture medium is generally "serum free", which means that the medium is essentially free of serum from any mammalian source (e.g. fetal bovine serum [FBS]). By "essentially free" is meant that the cell culture medium comprises between about 0-5% serum, preferably between about 0-1% serum and most preferably between about 0-0.1% serum (v/v).

A "mitogenic agent" or "growth factor" is a molecule which stimulates mitosis of the particular cell type under investigation. Generally, the mitogenic agent or growth factor enhances survival and/or proliferation of the cell type in cell culture. Examples of mitogenic agents include R5e/Ax1 receptor activators; activators of one or more members of the *erb*B receptor family; agents which elevate cAMP levels in the culture medium (*e.g.* forskolin, cholera toxin, cAMP or analogues thereof); adhesion molecules such as neural cell adhesion molecule (N-CAM), laminin or fibronection; progesterone; neurotrophic factors such as brain-derived neurotrophic factor (BDNF) and ciliary neuronotrophic factor (CNTF); neurotrophin-3, -4, -5, or -6 (NT-3, NT-4, NT-5. or NT-6); or a nerve growth factor such as NGF-β; platelet-derived growth factor (PDGF); epidermal growth factor (EGF); fibroblast growth factor such as acidic FGF (aFGF) and basic FGF (bFGF): transforming growth factor (TGF) such as TGF-α and TGF-β, including TGF-β1, TGF-β2, TGF-β3, TGF-β4, or TGF-β5: insulin-like growth factors, including IGF-I, IGF-II and des(1-3)-IGF-I (brain IGF-I): insulin-like growth factor binding proteins such as IGFBP-1, -2, -3, -4, -5, or -6: and hormones such as estrogen. testosterone, progesterone. thyroid hormone. and insulin.

"Pharmaceutically acceptable" carriers or vehicles are ones which are nontoxic to the mammal being exposed thereto at the dosages and concentrations employed. Often the pharmaceutically acceptable carrier is an aqueous pH buffered solution. Examples of pharmaceutically acceptable carriers include buffers such as phosphate, citrate, and other organic acids: antioxidants including ascorbic acid; low molecular weight (less than about 10 residues) polypeptides; proteins, such as serum albumin, gelatin, or immunoglobulins; hydrophilic polymers such as polyvinylpyrrolidone: amino acids such as glycine, glutamine, asparagine. arginine or lysine: monosaccharides, disaccharides and other carbohydrates including glucose, mannose, or dextrins; chelating agents such as EDTA; sugar alcohols such as mannitol or sorbitol: salt-forming counterions such as sodium; and/or nonionic surfactants such as Tween, Pluronics or polyethylene glycol (PEG). Pharmaceutically acceptable carriers or vehicles also include semi-solid, gelatinous or viscous support medium. Examples of such gelatinous carriers or vehicles include collagen, collagen-glycosaminoglycan, fibrin, polyvinyl chloride, polyamino acids such as polylysine or polyomithine, hydrogels, agarose, dextran sulfate and silicone. Pharmaceutically acceptable vehicles appropriate for the transplantation of developing nerve cells are found in, for example International Publication No. WO 92/03536.

A nervous system disease or disorder is meant to include traumatic lesions (*e*.*g*. caused by physical injury or surgery, and compression injuries); ischemic lesions (*e*.*g*. cerebral or spinal cord infarction and ischemia); malignant lesions; infectious lesions (*e*.*g*. resulting from an abscess or associated with infection by human immunodeficiency virus, Lyme disease, tuberculosis, syphilis, or herpes infection); degenerative lesions (*e.g.* associated with Parkinson's disease, Alzheimer's disease, Huntington's chorea or amyotrophic lateral sclerosis); lesions associated with nutritional diseases or disorders (*e.g*. Vitamin B12 deficiency, folic acid deficiency, Wernicke disease, tobacco-alcohol amblyopia, Marchiafava-Bignami disease and alcoholic cerebellar degeneration); neurological lesions associated with systemic diseases (*e*.*g*. associated with diabetes, systemic lupus erythematosus, carcinoma or sarcoidosis); lesions caused by toxic substances (*e*.*g*. alcohol, lead or neurotoxin); and demyelinated lesions (*e*.*g*. associated with multiple sclerosis, human immunodeficiency virus-associated myelopathy, transverse myelopathy of various etiologies, progressive multifocal leukoencephalopathy and central pontine myelinolysis).

The term "treatment" within the instant invention is meant to include therapeutic treatment, as well as prophylactic, or suppressive measures for a disease or disorder. Thus, for example, in the case of Alzheimer's disease, successful administration of an agent prior to onset or during the course of the disease results in "treatment" of the disease whether or not associated with delayed or prevented onset of the disease ifthe administration is associated with an apparent clinical benefit. Such a benefit may be any benefit including but not limited to the lessening of a side effect of another therapy or the disappearance of a symptom. For example, successful administration of the agent after clinical manifestation of the disease to combat the symptoms of the disease comprises "treatment" of the disease. "Treatment" also encompasses administration of the agent after the appearance of the disease in order to eradicate the disease. Successful administration of the agent after onset and after clinical symptoms have developed, with possible abatement of clinical symptoms and perhaps amelioration of the disease, also comprises "treatment" of the disease.

The term "mammal" refers to any non-human mammal classified as a mammal, including non-human primates, cows, horses, dogs, sheep and cats.

### Modes for Carrying out the Invention

It will be recognized by the skilled artisan that for the in vitro culture of the neural epithelial precursor cells of the present invention routine aseptic cell culture techniques are to be employed along with the methods of the present invention. The invention will now be described with reference to these and other cell culture techniques.

For the isolation of the normal neural epithelial precursor cells (NFP cells) of the present invention mammalian neural tissue is obtained from a non-human mammal in the zero-somite (0-somite) stage of development. This stage of the developing embryo is characterized by the appearance of the neural groove, the developing allantois and amnion formation. The stage precedes the appearance of the first somites and is generally identified by the appearance of the neural plate. According to the present invention, this stage is developmentally equivalent to the ninth day of rat embryonic development. Therefore, according to the present invention embryonic day nine rat neural tissue or developmentally equivalent neural tissue in other non-human mammalian species is employed. According to a preferred aspect of the invention, embryonic neural tissue at the ninth day of rat embryonic development between the hours of 3 p.m. and 4 p.m. or developmentally equivalent neural tissue from another mammalian species is employed.

One skilled in the art will recognize that mammalian embryos follow similar developmental patterns that can be identified as distinct morphological stages. The stages are identified broadly by day of appearance of the particular morphological feature, such as the neural plate. The timing of appearance of principle morphological features, however, varies between species. Accordingly, embryonic neural tissue is isolated just prior to the appearance of the first somites. This stage is commonly referred to as the "zero-somite" or "0-somite" stage of development. In the rat species the first somites appear between the 9th and 10th day of embryonic development and developing neural tissue is obtained at the ninth day of development just prior to the appearance of the first somites. The rat species anticipates the murine species developmentally by 1.5 to 2 days for the appearance of the same principal features. Accordingly, in the mouse species, the first somites appear at about the eighth day of embryonic development. Therefore, for the.murine species, neural tissue is generally obtained at about between the sixth to about the eighth day of murine embryonic development.

Neural tissue obtained from the embryo developmentally equivalent to the E9 rat embryo is generally isolated from the developing embryo by dissection employing techniques commonly available to the skilled artisan. One skilled in the art of mammalian developmental biology will be able to identify and isolate the neural tissue using techniques that are standard in the art. Generally microscopic dissection is employed to separate the caudal portion of the embryo, followed by removal of the developing mesoderm and endoderm. According to the present invention the remaining neural tissue is employed and preferably the neural tube is employed.

Neural tissue and preferably neural tube from the developing embryo at rat E9 or the developmentally equivalent stage is cultured in the presence of embryonic Schwann cells or advantageously in the presence of culture media obtained from the in vitro culture of embryonic Schwann cells. Embryonic Schwann cells are isolated by the methods described herein or by methods known to those skilled in the art. The embryonic Schwann cells may be a primary or secondary culture or a continuously propagated line as described herein. Additionally, continuous embryonic Schwann cell lines obtained by virtue of viral transformation or oncogene transfection may be employed as a source of embryonic Schwann cells or embryonic Schwann cell conditioned media. Alternatively, the embryonic Schwann cells may be a Schwannoma as described by (Ansselin and Corbeil (1995) In Vitro Cell Dev. Biol. 31:253-254; Brockes et al. (1979) Brain Res. 277:389-392; Morrissey et al. (1991) J. Neurosci. 11(8):2433-2442; Peulve et al. (1994) Exp. Cell. Res. 214:543-550; Rutkowski et al. (1992) Ann. Neurol. 31:580-586; Watabe et al. (1990) J. Neuropathol. Exp. Neurol. 49:455-467) for example. Embryonic Schwann cells and methods of obtaining them are known to those skilled in the art (Messing et al. (1994) J. Neurosci. 14:3533-3539). Copending U.S. Application Serial No. 08/435,436 filed May 10, 1995 describes an exemplary method of obtaining embryonic Schwann cell cultures.

In obtaining Schwann cells or in selecting nerve tissue which comprises Schwann cells (*e*.*g*. peripheral nerve tissue) for isolation of the Schwann cells it is important that the cells be derived from an embryonic non-human mammal. According to the present invention, an embryonic Schwann cell is obtained from a non-human mammal at a stage developmentally equivalent to rat day 14 stage of embryonic development or at any stage thereafter through the last day of embryonic development. Preferably, Schwann cells developmentally equivalent to rat embryonic day 15 are employed. Appropriate tissue sources include, for example, dorsal root ganglia

In a preferred aspect of the present invention, the mammalian species from which the Schwann cells are derived matches the species from which the normal NEP cells are isolated. However, species matching is not an absolute requirement. For example, rat embryonic Schwann cells may be employed as the embryonic Schwann cell source for the isolation of murine normal NEP cells in addition to rat normal NEP cells.

In an exemplary method of isolating embryonic Schwann cells, nervous tissue from a developing embryo (generally between rat day 14 and day 20) is obtained. A suitable size for the starting nerve tissue is generally between about 1mg to 1gm. The nerve tissue can be stored in medium (*e.g.* RPMI-1640 [Sigma], Liebovitz's L15 or Belzer's UW solution), prior to culture. Pre-incubation of the embryonic Schwann cell may be advantageous if the tissue contains myelinated nerves to facilitate demyelination of the Schwann cells. For the pre-incubation of embryonic Schwann cells, the nerve tissue is desirably treated with one or more protease enzymes for a sufficient period of time to loosen connective tissue and thereby promote demyelination. Many protease enzymes are commercially available which can be used for this step and include collagenase, dispase and other serine proteases, for example. Excess enzyme can be removed by gentle washing with culture medium.

Embryonic Schwann cell culture is typically carried out on a solid phase (*e.g.* a plastic tissue culture dish or plate) coated with extracellular matrix/adhesion proteins such as laminin, fibronectin, poly-lysine or collagen, with laminin being preferred. This allows preferential adhesion and migration of the Schwann cells onto the coated solid phase. The Schwann cells are cultured in the laminin-coated culture plates in a suitable culture medium.

Culture media for the culture of embryonic Schwann cells are well known to persons skilled in the art and include, but are not limited to, commercially available media such as F12/ DME, Ham's F10 (Sigma), Minimal Essential Medium ([MEM], Sigma), RPMI-1640 (Sigma), and Dulbecco's Modified Eagle's Medium ([DMEM], Sigma). Any of these media may be supplemented as necessary with, ions (such as sodium, chloride, calcium, magnesium, and phosphate), buffers (such as HEPES), nucleosides (such as adenosine and thymidine), trace elements (defined as inorganic compounds usually present at final concentrations in the micromolar range), and glucose or an equivalent energy source. Other supplements may also be included at appropriate concentrations that would be known to those skilled in the art. The preferred culture medium is F12/DME (1:1) containing 4.5 g/liter glucose supplemented with 15 mM HEPES, pH 7.4, 1.2 g/liter sodium bicarbonate.

According to the present invention, whereas the medium may be supplemented as described above to promote the growth and expansion of the embryonic Schwann cells in vitro, for the purposes of the present invention, serum free culture conditions are employed. The embryonic Schwann cells are preferably placed in dishes (*e.g.* 100mm petri dishes) in culture medium supplemented with suitable mitogenic agents. For example, for the rat embryonic Schwann cell the culture medium is preferably serum free medium which is supplemented with one or more mitogenic agents including, at least an *erb*B activator (*e*.*g*. heregulin), insulin (or an IGF), bovine pituitary extract (Roberts et al., (1990) Am. J. Physiol. 3:415-425), and a stimulator of cAMP production (e.g. forskolin) in the appropriate ranges to promote growth and expansion of the embryonic Schwann cells and avoiding the use of growth factors such as acidic or basic fibroblast growth factor, epidermal growth factor, and the like.

Therefore, in an exemplary method of isolating rat embryonic Schwann cells for co-culture according to the present invention dorsal root ganglia from E14 rat embryos or the developmentally equivalent embryo from other mammalian sources is isolated. Clean dorsal root ganglia are incubated with collagenase/dispase (Boehringer Mannheim Cat. No. 1097113) at a concentration of 0.3% for about 45 minutes. The dorsal root ganglia is then rinsed clean and dispersed by gentle pipetting with a 1 ml Pipetman™. The dispersed cells are collected by centrifugation (1000 rpm, 5 min) washed three times with F12/DMEM and plated on laminin coated plates in F12/DMEM supplemented with recombinant human insulin (5µg/ml), transferrin (10µg/ml), progesterone (2x10⁻⁸ M), bovine pituitary extract (Roberts et al. supra)(10µg/ml), recombinant heregulin (3 nM, HRG-ß1₁₇₇₋₂₄₄) (Holmes et al. [1992] Science 256:1205-1210), forskolin (5 µM) and α-tocopherol (5 µg/ml). Schwann cells grow to a confluent monolayer after 4 days.

Schwann cells from the primary cultures grown on the laminin substrate are passaged every 4 days at a 1:4 split ratio onto laminin-coated dishes in medium supplemented with progesterone, insulin, α-tocopherol, heregulin, forskolin, and transferrin, as well as bovine pituitary extract. Culture media from the culture of embryonic Schwann cells is obtained from embryonic Schwann cell cultures at any time after the cells have reached confluency. Preferably Schwann cell conditioned media is collected after about 2-4 days and most preferably after about 4 days of culture.

For the isolation of embryonic neural epithelial precursor cells by co-culture with embryonic Schwann cells or embryonic Schwann cell conditioned medium, dispersed neural tissue obtained as described above from the rat E9 embryo or the developmentally equivalent non-human mammalian source is placed in culture with embryonic Schwann cells (ESC's) or in culture medium supplemented with embryonic Schwann cell conditioned medium (ESCCM). Co-culture of the neural tissue with the ESCs may occur by virtue of the cells being placed together in the same culture vessel, as well as by co-culture where the cells, i.e., ECS's and the cells of the developing neural tube, are separated by a physical barrier which allows the transport of soluble factors across a semipermeable membrane while avoiding physical contact of the cells themselves.

According to the present invention an appropriate culture vessel is previously coated with a suitable extracellular matrix protein such as laminin. Appropriate culture vessels for the isolation and culture of neural epithelial precursor cells of the present invention include glass and plastic vessels. Although glass vessels may be employed, generally the solid phase is a plastic or polystyrene tissue culture dish or plate such as those routinely employed for mammalian cell culture. The tissue culture vessel is selected keeping in mind that a small quantity of cells is initially cultured. Generally, between about 100 and 1 X 10⁵ are plated onto or into the vessel. The vessel is selected so that the cells remain in contact in a small, generally about 100 to 200 microliter volume. Appropriate tissue culture vessels include plastic 96 well plates.

In addition the plastic plate or tissue culture vessel is normally coated with an extracellular matrix or attachment factor that contains some or all of the components of the extracellular matrix that most cells of the developing vertebrate organism are in contact with during normal development. Collagen and laminin are generally produced by the developing mammalian neuroblast. Therefore, extracellular matrix/adhesion proteins such as collagens, glycosaminoglycans, proteoglycans, and glycoproteins are employed. Most preferred are the glycoproteins such as fibronectin, laminin, enatactin, and hyaluronectin. Most preferred according to the present invention is laminin.

The medium used in the co-culture isolation of the neural epithelial cells of the present invention may be any of a number of synthetic basal medium that are commercially available. The medium is selected bearing in mind that basal media differ in their survival, growth, and differentiation promoting properties. Suitable culture media include, but are not limited to. commercially available media such as F12/ DME. Ham's F10 (Sigma). Minimal Essential Medium ([MEM], Sigma), RPMI-1640 (Sigma), and Dulbecco's Modified Eagle's Medium ([DMEM], Sigma). In addition, any of the media described in Ham and Wallace, Meth. Enz., $58:44 (1979), Barnes and Sato, Anal, Biochem., 102:255 (1980), U.S. 4,767,704; 4,657,866; 4,927,762; or 4,560,655; WO 90/03430; WO 87/00195; U.S. Pat. Re. 30.985; or U.S. 5,122,469, the disclosures of all of which are incorporated herein by reference, may be used as a basal culture medium. The preferred culture medium is F12/DME (1:1).

These media and especially F12/DME may be supplemented with ions (such as sodium, chloride. calcium. magnesium. and phosphate), buffers (such as HEPES), nucleosides (such as adenosine and thymidine), trace elements (defined as inorganic compounds usually present at final concentrations in the micromolar range), and glucose or an equivalent energy source.

According to the present invention, for the isolation of neural epithelial precursor cells from co-culture with embryonic Schwann cells or embryonic Schwann cell conditioned medium, the basal medium described above is additionally supplemented with bovine pituitary extract (Roberts et al., (1990) Am. J. Physiol. 3:415-425) at about 0.01 to about 100 µl/ml and preferably at about 3-5 µl/ml. An agent which elevates cAMP levels in the medium is additionally desirably present. Forskolin is the preferred such agent and suitable concentrations of this molecule are in the range from 0.1 µM to 50 µM forskolin, more preferably 1 µM to 20µM forskolin, and most preferably about 5 *µ*M forskolin. Insulin or an IGF (*e.g.* IGF-I or IGF-II) is also desirable for the co-culture. Preferably, insulin will be added to the medium in the range 0.01 µg/ml to 100µg/ml, more preferably 1µg/ml to 10µg/ml, and most preferably 10µg/ml. Additionally, the culture media may optionally include a mitogenic agent such as a molecule which activates a member of the *erb*B receptor family. Heregulin is the preferred activator and the human heregulin-β1₁₁₇₋₂₄₄ fragment is the most preferred mitogenic agent (Holmes et al., (1990) Science, 256:1205-1210). The concentration of heregulin in the medium will preferably be in the range of about 0.001nM to 10nM, more preferably 0.1nM to 10nM, and most preferably 1nM to 10nM. Progesterone may also be added to the culture medium in the range of about 0.1nM to 200nM, and more preferably about 1nM to 100nM, and most preferably about 3nM. An iron source such as transferrin may be employed. The concentration of transferrin in the medium will generally be in the range of about 0.1 µg/ml to about 100 µg/ml and preferably between about 5 µg/ml and 10 µg/ml. Other optional supplements include Vitamin E (an anti-oxidant and anti-transforming agent), preferably in the range 0.1 µg/ml to 100 µg/ml, more preferably 1 µg/ml to 20 µg/ml, and most preferably 5 µg/ml to 10 µg/ml; and chemically defined lipids (Sigma Cat # 11905-015), preferably in the range from about 1µL/ml to 500µL/ml, more preferably 10µL/ml to 100µL/ml, and most preferably 25µL/ml to 50µL/ml.

A preferred media formulation according to the present invention includes recombinant human insulin at about 5 µg/ml, progesterone at about 2x 10⁻⁸ M, bovine pituitary extract (Roberts et al., (1990) Am. J. Physiol. 3:415-425) at about 10µg/ml, recombinant heregulin at about 3 nM (HRG-ß1₁₁₇₋₂₄₄ (Holmes et al. [1992] Science 256:1205-1210), forskolin at about 5 µM, transferrin at about 5 µg/ml and α-tocopherol at about 5 µg/ml.

Culture conditions, such as temperature, pH. dissolved oxygen (dO₂) and the like, are those generally used in mammalian cell culture and will be apparent to the ordinarily skilled artisan. Generally, the pH is adjusted to a level between about 6.5 and 7.5 using either an acid (e.g., CO₂) or a base (e.g., Na₂CO₃ or NaOH). A suitable temperature range for culturing mammalian cells is between about 30 to 38°C and a suitable dO₂ is between 5-90% of air saturation.

Advantageously, the cells of the present invention may be co-cultured and isolated with embryonic Schwann cell conditioned medium in place of the embryonic Schwann cells. According to this aspect of the present invention, the media described above is supplemented with culture media obtained from the culture of embryonic Schwann cells. Preferably, such conditioned media is obtained from the culture of embryonic Schwann cells after about 1 to 4 days post-initiation of an embryonic Schwann cell culture. Minimally, the embryonic Schwann cells should have reached confluency prior to harvesting the culture media. Preferably, the culture supernatant from the embryonic Schwann cells is harvested by removal of the cells. This is generally accomplished by centrifugation and/or filtration. The remaining culture media is used as a media supplement.

Embryonic Schwann cell conditioned media obtained in this manner can be used as a media supplement as obtained, or it may first be concentrated. Concentration is generally accomplished keeping in mind that the concentration devise should allow for the retention of essentially all proteinaceous substances. This generally means using a device with a low protein binding molecular weight cutoff of about between 3000 and 5000 kilodaltons. Concentration advantageously allows for smaller volume additions to the media described.

The amount of embryonic Schwann cell conditioned media (ESCCM) employed as a media supplement is not a fixed amount but varies with the batch of conditioned media employed. The ESCCM should be present in at least an amount sufficient to promote the growth of the neural epithelial cells of the present invention. Embryonic Schwann cell conditioned media is added at a ratio of about 1:1 to about 10:1 parts of the media formulation described above to the embryonic Schwann cell conditioned media and preferably at about 5 parts of the media formulation described above to about 1 part ESCCM. With ESCCM concentrated prior to addition as a media supplement the amount of ESCCM media added as a supplement varies according to the concentration. For ESCCM concentrated 10 fold, the ESCCM is added to the basal media in the range of 1% to about 50% (v/v) and preferably about 30% (v/v).

When the neural tissue is placed in co-culture with the embryonic Schwann cells it is not necessary to supplement the basal media with embryonic Schwann cell conditioned medium. Neural tissue is plated onto plates precoated with the appropriate extracellular matrix to which embryonic Schwann cells have been added. The number of embryonic Schwann cells used in co-culture varies with the size of the culture vessel. An amount of embryonic Schwann cells sufficient to form a monolayer in the culture vessel is sufficient. Generally embryonic Schwann cells plated at an initial density of about 1 to about 5 x 10⁵ cells per cm² culture medium is sufficient.

Neural epithelial precursor cells obtained from the dispersed neural tissue as described above are plated keeping in mind that the cells require cell to cell contact in order to survive. Optimal cell growth is achieved at an initial plating density of about between 0.5 to about 5 x 10⁵ cells per ml.

Under these conditions neural epithelial precursor cells survive and proliferate to form large colonies of compacted monolayer epithelial cells containing some differentiated neurons bearing long processes.

After about 2-6 days in culture, and generally after about 4 days in culture, colonies of neural epithelial precursor cells obtained as described above are removed from the extracellular matrix protein coated plates and replated in the media as described above at a density of about 1-2 x 10⁵ cells/ml. The cells are subcultured by repeating this process for several cell passages. Generally about 5 to 6 passages are performed.

During this period the cultures contain two major cell types, one is the neural epithelial precursor cell type of the present invention and the other is a bipolar Schwann cell-like cell. The bipolar Schwann cells are removed from the culture by removing heregulin from the culture medium.

Following this, normal neural precursor cell lines are maintained in culture in media as described and preferably in F12/DME supplemented with forskolin, BPE. transferrin, progesterone, and α-tocopherol. Embryonic Schwann cells or embryonic Schwann cell conditioned media is generally only required to isolate the cell line of the present invention.

Cell lines isolated from embryos can be frozen for long term storage in serum free media containing 10% dimethylsulfoxide (DMSO) or glycerol.

### USE

Neural epithelial precursor cells produce a number of neurotrophic and neural growth-promoting factors (Placzek et al. (1993) Development 117:205-218; Ghosh and Greenberg (1995) Neuron 15:89-103)and themselves respond to a number of growth promoting substances. Accordingly, normal neural epithelial cells isolated and cultured using the techniques described herein can be used to produce and detect these factors. It is desirable to have populations of normal neural epithelial precursor cells in cell culture for isolation and detection of neural epithelial precursor cell specific factors such as sonic hedgehog (SHH) (Hynes, M. et al.,(1994) Neuron, 15:35-44). Such factors are useful as diagnostic tools themselves or can be used an antigens to generate antibodies for diagnostic use.

The cell lines themselves are useful for diagnostic purposes. For example, the cells of the present invention may be used to detect the presence of various growth promoting substances. According to this aspect of the present invention, the cells may be used to monitor the isolation of specific neural epithelial growth promoting factors during the course of an isolation procedure. Cell based assays can be simply devised utilizing the neural epithelial precursor cells of the present invention. For example, the synthesis of DNA and the incorporation of a radiolabelled nucleotide are commonly employed in cell based assays for determining the responsiveness to a particular sample containing a growth promoting substance. Such cellular assays may be diagnostically relevant for the detection of the presence or absence of clinically relevant substances or in the course of a therapeutic treatment.

Alternatively, the normal neural epithelial cells may be used for the recombinant expression and production of various proteins. According to this aspect of the invention the cell lines isolated by means of the present invention may be transfected with an expression vector containing a nucleic acid sequence encoding a protein of choice. One ofordinary skill in the art will choose an appropriate DNA expression system for use in association with the cell lines of the present invention. Proteins produced in this manner advantageously possess the particular glycosylation patterns characteristic of the neural epithelial precursor cell.

It is also beneficial to have populations of non-human mammalian NEP cells for use in cell based therapies. The NEP cells of the present invention may be used alone or in combination with other cell types for direct transplantation into areas of damaged neural tissue for the treatment of diseases or disorders of the nervous system. The cells alone or in combination with other cell types provide direct replacement and auxiliary support for the regeneration of or repopulation of a nervous system lesion. According to this aspect of the invention the normal neural epithelial cells are formulated in an appropriate pharmaceutical vehicle as described herein. Isolated NEP cells, pharmaceutical compositions comprising NEP cells, or prostheses filled with the NEP cells can be introduced into a nervous system lesion using any method known in the art for the treatment of neurological diseases such as those mentioned above and especially, neurodegenerative diseases or disorders including degenerative lesions associated with Parkinson's disease, Alzheimer's disease, Huntington's chorea or amyotrophic lateral sclerosis.

The normal neural epithelial cells of the present invention may be used to repopulate areas of neuronal damage or degeneration. This type of cell based therapy is analogous to the unilateral transplantation of human fetal mesencephalic tissue into the caudate nucleus of patients with Parkinson's disease (Spencer et al., (1992) New Engld. J. Med. 327:1541-1548). Alzeheimer's disease and other disorders such as Down's syndrome, post encephalitic Parkinsonism, dementia pugistica, are characterized by neuronal cell loss and changes in neuronal morphology (Goldman, and Côté (199) "Aging of the Brain."). The neural epithelial precursor cells ofthe present invention which are capable of in vivo differentiation and integration can be used to repopulate specific areas of damaged neuronal tissue. Methods for delivering cells to the brain are generally known and described in International Publication No. WO 90/06757 and WO 93/14790.

Neural transplantation or "grafting" involves transplantation of the neural epithelial precursor cells into or onto the central nervous system of a host or recipient. These procedures include the transplantation of the neural epithelial precursor cells within a host brain, such as intraparenchymal transplantation, as well as the deposition of the cells on the surface of the host brain in a gelatinous vehicle such as those described below. Intraparenchymal transplantation can be accomplished by injecting the neural epithelial precursor cells of the invention into a host or recipient brain parenchyma. Procedures for the transplantation of cells into selected regions of a host brain are generally known and include those described in International Publication No. WO 90/06757 as well as the references cited therein.

For grafting, generally, the isolated neural epithelial precursor cells are suspended in 5 phamaceutically acceptable vehicle such a balanced glucose-saline solution and injected into a predetermined region of the host brain using a sterile microsyringe. Therefore, the present invention allows for a method of transplanting cells comprising isolating a normal neural epithelial cell type, and injecting or grafting the normal neural epithelial cell into or onto a recipient brain.

In a particular embodiment, the normal neural epithelial cells may be used to produce neural epithelial precursor cell specific proteins for delivery of endogenous neurotrophic and neural growth promoting factors in vivo. Alternatively, a variety of methods are currently available to genetically modify cells of neural origin for site specific delivery of biologically active proteins. For example, in Alzheimer's disease, a deficiency of acetylcholine can be corrected by delivering the acetylcholine using the present cell line as a vehicle for the in situ expression of the neurotransmitter. Recently, neural precursor cells have been used as a carrier for nerve growth factor in the brain (Martinez-Serrono (1995) Neuron 15:473-484). For example, it may be possible to introduce a gene encoding a specific molecule which is known to promote nerve fiber growth, or a gene for a specific growth factor molecule believed to sustain or promote neuronal health. The neural epithelial precursor cells may be transfected or infected with a DNA sequence encoding a biologically active protein such as mammalian nerve growth factor or neurotransmitter and subsequently used for increased localized production of the protein. Cell and site specific expression of specific molecules will generally result in local production of prophylactically and therapeutically effective amounts of the desired biologically, active protein. Methods for transplanting cells which may or may not express a transfected gene product in the brain are provided generally in International Publication No. WO 93/14790 and WO 93/10234.

By way of example and not of limitation, a recombinant retroviral vector comprising a DNA sequence encoding a biologically relevant protein is utilized to directly infect the neural epithelial precursor cells of the present invention in vitro. The infected cells can then be delivered to the specific tissue target site utilizing methods known in the art including but not limited to techniques which are commonly employed to repopulate areas of suspected neuronal damage. Any number of retroviral constructs which express a biologically active protein may be utilized by the skilled artisan according to this aspect of the invention.

As used herein, the term "pharmaceutically acceptable" generally means approved by a regulatory agency of the Federal or a state government or listed in the U.S. Pharmacopeia or other generally recognized pharmacopeia for use in animals, and more particularly in humans. Pharmaceutical formulations comprising isolated NEP cells and a pharmaceutically acceptable carrier or vehicle as described herein can be prepared using techniques which are well known in the art. Suitable formulations include biological buffers, such as phosphate buffered saline, saline, Dulbecco's Media, and the like. The formulation of choice can be accomplished using a variety of the aforementioned buffers, or even excipients including, for example, pharmaceutical grades of glucose, mannitol lactose, starch, magnesium stearate, sodium saccharin cellulose, magnesium carbonate, and the like. Optionally, the pharmaceutical formulation may include one or more mitogenic agents and other components *(e.g.* extracellular matrix proteins such as laminin). If a gelatinous support is used as the pharmaceutically acceptable vehicle, the NEP cells may be introduced into a liquid phase of the vehicle which can be treated such that it becomes more solid (*e*.*g*. a unpolymerized vehicle may be induced to polymerize). For example, the NEP cells may be added after collagen solubilized in acetic acid in H₂O is brought to neutral pH, by addition of a suitable base such as NaOH and to isotonicity by the addition of salts.

The NEP cells can also be delivered in prostheses or devices such as those which have been described in the literature. Generally, a solid porous tube filled with the NEP cells (preferably formulated in a gelatinous vehicle) is used as the prosthesis.

The following examples are offered by way of illustration and not by way of limitation. The disclosures of all citations in the specification are expressly incorporated herein by reference.

### EXAMPLES

### EXAMPLE 1

### Introduction

Given their developmental potential. neuroepithelial cells from embryonic day 9,0-somite rat embryos were grown in a serum free hormone supplemented medium. Here the isolation, establishment and characterization of a neuroepithelial precursor cell line which can be induced to differentiate into neural cell types in vitro and in vivo is demonstrated.

### Methods

### A. Neuroepithelial cell growth:

E9 rat neural tubes (0-somite) were dissected under a dissecting microscope using fine needles. The caudal portion was removed by cutting through the middle of Hassen's node. Mesoderm and endoderm were removed after a brief incubation with collagenase and dispase (Boehringer Mannheim, 0.2%. 10 min at 0 C). The clean neural tubes were washed 5x by transfer from plate to plate of F12/DMEM with 1% BA. The neural tubes were then dispersed into small aggregates (preferentially 20 to 50 cells in each aggregate) by gentle pipetting with yellow tips on a 200µl Pipetman™ pipetter. Complete dispersion should be avoided. The dispersed cells were plated in 96 well multiplates (at a density of 20 neural tubes divided into 96 wells) precoated with attachment factors in serum free medium supplemented with different combinations of growth factors. Good cell survival and growth was found only in conditions which contained bovine pituitary extract (Roberts et al 1989 supra), insulin, forskolin and either in co-culture with the embryonic Schwann cell line or with concentrated Schwann cell conditioned medium (ESCCM). In these conditions, some precursor cells survived and proliferated to form large colonies of compacted monolayer epithelial cells containing some differentiated neurons bearing long processes. Cells in other conditions died within 4 days in culture.

### B. Long term culture

Colonies of epithelial cells formed in the primary culture in F12/DME supplemented with 7F (forskolin (about 5 µM), BPE (about 10 µg/ml), insulin (about 5 µg/ml), transferrin (about 5 µg/ml), heregulin (about 3 nM), progesterone (about 2 x 10⁻⁸M), α-tocopherol (about 5µg/ml)) and ESCCM, were removed from the substrate with 0.2% collagenase/dispase at 37° C. The cells were washed free of the enzymes by centrifugation on a layer of 3% BA and plated onto laminin coated 24 well plates with F12 / DME supplemented with 7F. The cells were routinely subcultured by the same procedure for 5-6 passages. During this period the cultures contain two major cell types, one is the compact epithelial cells, the other is bipolar cells resembling Schwann cells or radial glia which were subsequently removed from the culture by removing heregulin from the culture medium and allowing the epithetial cells to grow at a higher density. The cells were then carried in 6F medium (7F medium without heregulin) on laminin coated plates and passaged every week using a 1 to 4 split.

### C. Cell proliferation studies

Cells between passages 25 and 35 were used in the experiments described below. Cells were routinely detached from the culture vessels and plated at the density indicated in laminin coated 24 well coated plates in F12/DME medium with different concentration of the growth factor to be tested. Alternatively the cells were plated in 6F for 24 hours and then the growth factor being studied was added at different concentrations in the presence of all other growth factors in 6F. Cell numbers were counted with Coulter counter after complete dispersion ofthe cells with trypsin-EDTA (Gibco BRL) at 37° C at least for 15 min.

### D. Cell differentiation studies:

Cells were plated in 6F (Insulin (5 *µ*g/ml), transferrin (5*µ*g/ml), α-tocopherol (5 *µ*g/ml), progesterone (2 x 10⁻⁸m), forskolin (5*µ*M), and bovine pituitary extract (10 *µ*g/ml)) at 1:8 split in laminin coated 24 well plates with F12/DME supplemented with 6F for 48 hours and then washed with F12/DME and refed with fresh medium supplemented with insulin, transferrin and α-tocopherol. Growth factors such as bFGF (recombinant human bFGF, Gibco BRL) aFGF (recombinant human aFGF, Gibco BRL), forskolin (Calbiochem), and/or NGF(7.5 S mouse NGF. Collaborative Research Inc.) were added individually or in combinations, as indicated. Medium was changed once after a 48 hour incubation with the respective factors. Cultures were fixed for unmunocytochemistry at the indicated time with ice cold 1% glutaraldehyde (Ted Pella. Inc.) in a 3% sucrose solution on ice for 30 minutes. The fixed cells were rinsed with ice cold milli-Q water, treated with methanol containing 3% H₂O₂ for 30 minutes at room temperature and washed with PBS. The cells were then incubated with PBS containing 0.2% TRITON X-100™ detergent, 5% goat serum and 0.1 M NH₄HCO₃ for I hour at 37° C. Primary antibodies were diluted in dilution buffer (PBS with 0.1% TRITON X-100™ detergent and 1% BA) and were added at 250 µl/well. Incubation with primary antibodies was carried out at 4° C overnight. Excess primary antibody was washed 5x with the dilution buffer and the cells were then incubated with appropriate enzyme conjugated secondary antibodies (Anti-mouse Ig F(ab)'-alkalinephosphatase, Anti-mouse Ig F(ab)'-peroxidase and anti-rabbit IgG F(ab)'-peroxidase were purchased from Boehringer Mannheim) at 37° C for 60 min. The cells were washed 5x with dilution buffer and 2x with either 0.05 M sodium acetate pH 5.0 for peroxidase conjugates or with phosphatase substrate buffer. Specifically bound peroxidase activity was detected with DAB-H₂O₂ solution made using the Sigma peroxidase substrate kit. Alkaline phosphatase was detected with Boehringer alkalinephosphatase substrate (NTB/BCIP) diluted in substrate buffer. The color developing reaction was stopped by thoroughly rinsing with tap water and the specimens were preserve in glycerol/PBS (50:50). The stained samples were observed and micrographs were taken using a Nikon-overted bright field microscope.

### E. Immunofluorescence

For nestin immunofluorescence, cells grown on chamber slides were fixed in situ with 4% paraformaldehyde in phosphate buffer PH 7. for 30 minutes at room temperature. The fixed cells were washed 3x with PBS. blocked with 5% goat serum in PBS plus -0.2% TRITON X-100™ detergent at 37° C for 60 minutes. Anti-nestin antiserum raised in rabbit against the last 1200 amino acid in the rat nestin sequence. The antiserum was diluted at 1:1000 with dilution buffer (and incubated with the cells at 4° C overnight). After 5 washes with dilution buffer (PBS with 0.1% TRITON X-100™ detergent and 1% BA), the samples were incubated with goat anti-rabbit lgG F(ab)'-FITC (Boehringer Mannheim) at 37° C for I hour. The slides were washed 5x with dilution buffer and sealed in 50% glycerol in PBS. Specific immunofluorescence was observed and micrographs were taken under NIKON™ epifluorescence microscope.

### F. Western Blotting

Approximately 10° cells were directly dissolved in 100 ul loading buffer and heated to 95° C for 5 min. The sample was chilled on ice and loaded onto a minigel (Novex precasted SDS gel, 4-20%). The sample was fractionated with SDS electrophoresis at 75 mA until the dye front moved near the end. Electric transfer to nitrocellulose membrane was carried out in Tris-glysine-methanol buffer at 100 V for 1 hour. The membrane was washed with water and was saturated by incubation with 1% BA at 37°C with gentle shaking. Incubation of primary antibodies (anti-nestin antiserum 1:1000, anti-neuron specific antiserum 1:1000) was carried out at 37°C for 2 hours. Excess primary antibody were washed away with three changes of incubation buffer, 10 minutes for each change. The membrane was then incubated with anti-rabbit lgG F(ab)'-peroxidase for 1 hour. Finally, the membrane was stained with DAB-H₂O₂ as described above for immunocytochemistry.

### Results

Neuroepithelial cells dissected from E9 rat embryos survived poorly in most serum free conditions. No cells survived beyond 4 days in conditions supplemented with EGF, FGFs, IGFs and neurotropins alone or in combination. Significant cell survival and proliferation was found only in those cells co-cultured with embryonic Schwann cells in the presence of Schwann cell growth medium which contained insulin, transferrin. α-tocopherol, progesterone, forskolin, bovine pituitary extract and recombinant human heregulin. Under these conditions, epithelial cells rapidly proliferated to form large cell colonies which pushed off the Schwann cells or grew underneath the Schwann cell monolayer. These cell colonies can form secondary colonies after subculture. Addition of bFGF to the secondary cultures induced the cells to extend long processes which morphologically resembled neuronal processes.

In order to achieve pure cells, embryonic Schwann cell conditioned medium (ESCCM) was tested, as well as cultures with trans-well inserts containing Schwann cells. This separates the Schwann cells from the neuroepithelial cells by a physical barrier but allows many Schwann cell secreted molecules to pass through. Both methods resulted in the survival and proliferation of a small proportion of neuroepithelial cells, which formed large colonies of epithelial cells with some differentiated neurons which extended long processes after 10 days. No cells survived in conditions without ESCCM or coculture. The cells grown in the presence of ESCCM were successfully subcultured. During the first 5 to 6 passages, the cultures comprised a mixture of cell types, epithelial type of cells, spindle-shaped Schwann cell like cell type and aggregated small neurons with long and fine processes. Secondary cultures no longer require the Schwann cell conditioned medium. The epithelial type of cells were enriched and become dominant in subsequent cultures by removing heregulin a potent glial cell mitogen and keeping the cells at a high density.

These normal neural epithelial precursor cells required cell-cell contact for survival and growth. Dispersion of the culture to single cells, even with a brief trypsin treatment, resulted in cell death. Optimal growth was achieved when cells were plated at density of 1-2 x 10⁵ cells/cm² A population doubling time of 50 hours was calculated, however the growth rate is non-linear with a marked increase in the doubling time as the cells get denser. Confluent culture reached a density 10⁶ cells/cm² but always remained as a monolayer. The cells have been grown continuously for over 40 passages (>80 population doublings), no obvious change has been found in cell morphology and growth profile and no obvious cell senescence has been observed. Cell morphology

The cell line showed monolayer epithelial cell morphology with a large nucleus/cytoplasm ratio during routine subculture as described. Most cells showed positive immunofluorescent staining for the neural precursor cell-specific intermediate filament protein, nestin. A western blot of solubilized cell membranes stained with anti-nestin antibody showed a single band at molecular weight 220 kilodaltons (Kd), consistent with the reported molecular weight of nestin. A small proportion (<5%)also showed positive staining for neuron specific enolase which had a molecular weight of 46 Kd in the western blot.

### Growth factor response

The NEP cell line required the presence of insulin, bovine pituitary extract, and forskolin (or cholera toxin or cAMP analogs) for survival and growth. Removal of one of these three growth factors resulted in a sharp decrease in cell number (Figure 1A). Optimal concentrations of these growth factor were determined to be 10 ug/ml insulin. 3-10 uM forskolin and 0.3% (v/v) bovine pituitary extract (Figure 1B and 1C). Insulin can also be replaced by insulin-like growth factors. The cells are more sensitive to IGF-I than to IGF-II (Figure 1D). Growth factors such as PDGF, EGF, heregulin, leukocyte inhibitory factor (LIF), hepatocyte growth factor (HGF) and neurotropins (NGF, NT-3 and BDNF) did not increase cell number (Figure 1E,F). Members of the TGFß family of growth factors had an inhibitory effect on cell growth (Figure 2A). Addition of TGFB1 resulted in significant cell death (Figure 2A). In the absence of forskolin and BPE, bFGF increased cell survival. While all the cells died in F12/DME supplemented with insulin only, with the addition of bFGF, a large proportion of cells survived as floating cell aggregates. However, addition of bFGF in the presence of 7F greatly inhibited cell proliferation, completely blocking cell growth beyond 24 hours of treatment. In contrast, aFGF showed little effect (Figure 2B).

### In vitro differentiation of the NEP cell line:

To test this hypothesis, cells were grown in the presence of 6F alone (insulin, transferrin, bovine pituitary extract, heregulin, progesterone, and a-tocopherol), 6F + bFGF, or 6F + bFGF + forskolin, for 48 hours and then fixed and immunostained for a variety of neuronal markers. The cells showed a marked change in morphology in the presence of bFGF, and a further evolution towards neuronal- like morphology in the presence of bFGF + forskolin. These morphological changes were accompanied by the increased expression of neural specific markers visualized by immunohistochemistry. The number of cells expressing and the extent of expression of both vimentin and tubutin β was increased by FGF and further increased by the combination of bFGF and forskolin. Specific neuronal markers, such as Map-2, peripherin and the 160 kd neurofilament protein (NF 160) were also induced as the cells became more differentiated morphologically, although these were expressed to differing extents and in different ways under the different conditions. For example, NF 160 is expressed only in a few cell/cell junctions in the control conditions. With FGF alone there is continued expression at the junctions and some cytoplasmic expression while in the FGF + forskolin conditions NF 160 can be seen on the cell surface and in the neural extensions as well as cytoplasmically. While Map2 is expressed by most cells in the presence of both factors, peripherin is present on only a few of the cells. Cells exposed to both factors express protein kinase c (Pkc), as well as the excitatiry amino acids glutamate and aspartate. Neuron specific enolase, synaptophysin, and Tau were also induced in some cells in the cultures. Low levels of the glial markers such as GFAP and Gal-c were induced in some cells some conditions. These results are summarized in Table I.

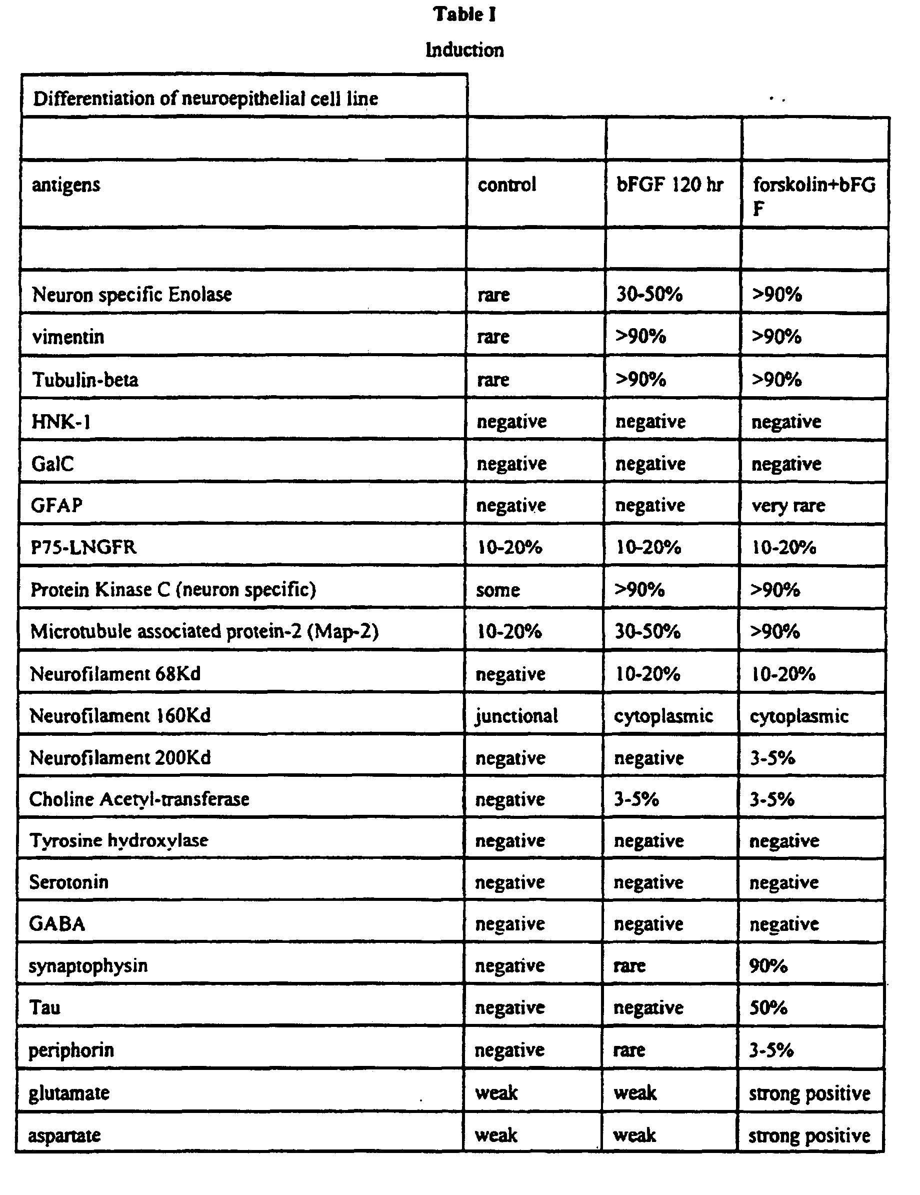

### EXAMPLE II

### In vivo injection and differentiation of NEP cells

NEP cells were isolated and expanded as described above. Approximately 1 x 10⁵ to 1 x 10⁶ cells were removed from culture and resuspended in a balanced saline solution containing glucose. The cells were injected via a sterile microsyringe into neonatal rat brain.

NEP cells for implantation were labeled with the fluorescent viable cell marker, PKH-26. When injected into neonatal rat brain, the NEP cells gave rise to several distinct neural cell phenotypes. Two major cell types were identified with typical morphology within the appropriate layer of the cerebellum. These cells had the morphology characteristic of Bergman glia and granule cells. In the hippocampus, most of the fluorescently labeled cells integrated into the dentate girus. The majority of these cells had a morphology typical of dentate granule cells. Other cells showed neuronal-like differentiation but could not be precisely categorized by morphology. In the cerebral cortex, bundles of cell processes span the ventricular region up to the surface of the cortex with terminal branches which resemble radial glial cell processes. Some of the cell bodies were traced and were located inside the lateral ventricle. Integrated cells were also found in left hemisphere, although cells were injected only in the right hemisphere of the brain.

The majority of the neurons differentiated from NEP in vitro in the presence of bFGF and forskolin displayed markers consistent with a cortical neuron identity. The differentiated neurons expressed a protein kinase C isoenzyme which is expressed exclusively in CNS cortical neurons. In addition, these neurons accumulated the excitatory amino acids, glutamate and aspartate, in their cytoplasm and in cell processes. These amino acids are used as neurotransmitters only in cortical neurons. The in vitro data is confirmed by the results of the in vivo experiments.

In the presence of bFGF and forskolin, most NEP cells differentiated into aminonergic neurons. However, a small number of cells began to express choline acety-transferase, a key enzyme involved in acetylcholine synthesis. The differentiation of cholinergic neurons in the NEP line was enhanced by addition of leukemia inhibiting factor (LIF). This result is consistent with the induction ofcholinergic neurons by LIF in neural crest cells and hippocampal cells. Although no mature glial cells were identified in vitro, the NEP cells can differentiate into Bergmann glial cells when injected into the cerebellum and radial glial cells in the cerebral cortex. Taken together, these data suggest that the NEP cell line is arrested at an early stage of neuroepithelial differentiation and maintains the ability to differentiate into several different neuronal and glial cells depending on the environment.

### Deposit of Materials

The following culture has been deposited with the American Type Culture Collection, 12301 Parklawn Drive, Rockville, MD, USA (ATCC):

| Cell Line | ATCC No. | Deposit Date |
|---|---|---|
| NEP95 | CRL 11993 | October 31, 1995 |

This deposit is being made under the provisions of the Budapest Treaty on the International Recognition of the Deposit of Microorganisms for the Purpose of Patent Procedure and the Regulations thereunder (Budapest Treaty). This assures maintenance of viable cultures for 30 years from the date of deposit. The cell line will be made available by ATCC under the terms of the Budapest Treaty, and subject to an agreement between Genentech, Inc. and ATCC, which assures permanent and unrestricted availability of the progeny of the culture to the public upon issuance of the pertinent U.S. patent.

The assignee of the present application agrees that if the culture on deposit should die or be lost or destroyed when cultivated under suitable conditions, it will be promptly replaced on notification with a viable specimen of the same culture. Availability of the deposited cells are not to be construed as a license to practice the invention in contravention of the rights granted under the authority of any government in accordance with its patent laws.

The foregoing written specification is considered to be sufficient to enable one skilled in the art to practice the invention. The present invention is not to be limited in scope by the culture deposited, since the deposited embodiments is intended as an illustration of an aspect of the invention and any cultures that are functionally equivalent are within the scope of this invention. The deposit of material herein does not constitute an admission that the written description herein contained is inadequate to enable the practice of any aspect of the invention, including the best mode thereof, nor is it to be construed as limiting the scope of the claims to the specific illustration that it represents.

## Claims

1. A method of isolating a normal neural epithelial precursor cell type comprising the steps of:
a) culturing neural tissue from a non-human mammalian embryo at the zero-somite stage of development in the presence of embryonic Schwann cells or embryonic Schwann cell conditioned media; and
b) isolating the normal neural epithelial precursor cells.

2. The method of claim wherein the neural tissue is neural tube.

3. The method of claim 2 wherein the neural tissue is cultured on a solid support precoated with an attachment factor.

4. The method of claim 3 wherein the attachment factor is laminin.

5. The method of claim 4 wherein the neural tissue is cultured in serum free media.

6. The method of claim 5 wherein the serum free media comprises bovine pituitary extract, insulin, and forskolin.

7. The method of claim 6₁ wherein the serum free media further comprises progesterone, and α-tocopherol.

8. The method of claim 2 wherein the neural tube is derived from a rat embryo.

9. The method of claim 8 wherein the neural tube is from a day 9 embryo.

10. The method according to claim 7 further comprising the step of separating the neural epithelial precursor cells from other cell types.

11. The method of claim 10 wherein the normal neural epithelial cells are subcultured in serum free medium lacking heregulin.

12. A normal neural epithelial precursor cell line isolated by the method of claim 1.

13. The normal neural epithelial precursor cell line of claim 13 which is a rat cell.

14. The normal neural epithelial cell line of claim 13 which is on deposit with the American Type Culture Collection and bears Accession Number CRL 11993.

15. A composition comprising the cell line of claim 12 and a pharmaceutically acceptable vehicle.

16. A composition comprising the cell line of claim 13 and a pharmaceutically acceptable vehicle.

17. A composition comprising the cell line of claim 14 and a pharmaceutically acceptable vehicle.

## Patentansprüche

1. Verfahren zum Isolieren eines normalen neuralen Epithelvorläuferzelltyps, umfassend die folgenden Schritte:
a) Kultivieren neuralen Gewebes aus einem nichtmenschlichen Säugetierembryo im Null-Somit-Entwicklungsstadium in Gegenwart von embryonalen Schwannschen Zellen oder von mit embryonalen Schwannschen Zellen konditioniertem Medium; und
b) lsolieren der normalen neuralen Epithelvorläuferzellen.

2. Verfahren nach Anspruch 1, worin das neurale Gewebe Neuralrohr ist.

3. Verfahren nach Anspruch 2, worin das neurale Gewebe auf einem festen Träger kultiviert wird, der mit einem Anlagerungsfaktor vorbeschichtet ist.

4. Verfahren nach Anspruch 3, worin der Anlagerungsfaktor Laminin ist.

5. Verfahren nach Anspruch 4, worin das neurale Gewebe in serumfreiem Medium kultiviert wird.

6. Verfahren nach Anspruch 5, worin das serumfreie Medium Rinderhypophysenextrakt, Insulin und Forskolin umfasst.

7. Verfahren nach Anspruch 6, worin das serumfreie Medium weiters Progesteron und α-Tocopherol umfasst.

8. Verfahren nach Anspruch 2, worin das Neuralrohr von einem Rattenembryo stammt.

9. Verfahren nach Anspruch 8, worin das Neuralrohr von einem 9 Tage alten Embryo stammt.

10. Verfahren nach Anspruch 7, das weiters den Schritt des Trennens der neuralen Epithelvorläuferzellen von anderen Zelltypen umfasst.

11. Verfahren nach Anspruch 10, worin die normalen neuralen Epithelzellen in serumfreiem Medium ohne Heregulin subkultiviert werden.

12. Normale neurale Epithelvorläuferzelllinie, isoliert durch das Verfahren nach Anspruch 1.

13. Normale neurale Epithelvorläuferzelllinie nach Anspruch 12, die eine Rattenzelle ist.

14. Normale neurale Epithelzelllinie nach Anspruch 13, die in der American Type Culture Collection hinterlegt ist und die Zugriffsnummer CRL 11.993 trägt.

15. Zusammensetzung, umfassend die Zelllinie nach Anspruch 12 und ein pharmazeutisch annehmbares Vehikel.

16. Zusammensetzung, umfassend die Zelllinie nach Anspruch 13 und ein pharmazeutisch annehmbares Vehikel.

17. Zusammensetzung, umfassend die Zelllinie nach Anspruch 14 und ein pharmazeutisch annehmbares Vehikel.

## Revendications

1. Procédé pour isoler un type de cellule précurseur épithéliale neurale normale, comprenant les étapes consistant :
a) à cultiver du tissu neural provenant d'un embryon de mammifère non humain au stade de somite zéro de développement en présence de cellules de Schwann embryonnaires ou d'un milieu conditionné avec des cellules de Schwann embryonnaires ; et
b) à isoler les cellules précurseurs épithéliales neurales normales.

2. Procédé suivant la revendication 1, dans lequel le tissu neural est le tube neural.

3. Procédé suivant la revendication 2, dans lequel le tissu neural est cultivé sur un support solide prérevêtu avec un facteur de fixation.

4. Procédé suivant la revendication 3, dans lequel le facteur de fixation est la laminine.

5. Procédé suivant la revendication 4, dans lequel le tissu neural est cultivé dans un milieu sans sérum.

6. Procédé suivant la revendication 5, dans lequel le milieu sans sérum comprend de l'extrait d'hypophyse bovine, de l'insuline et de la forskoline.

7. Procédé suivant la revendication 6, dans lequel le milieu sans sérum comprend en outre de la progestérone et de l'α-tocophérol.

8. Procédé suivant la revendication 2, dans lequel le tube neural est dérivé d'un embryon de rat.

9. Procédé suivant la-revendication 8, dans lequel le tube neural est dérivé d'un embryon au 9^{e} jour.

10. Procédé suivant la revendication 7, comprenant en outre l'étape de séparation des cellules précurseurs épithéliales neurales des autres types cellulaires.

11. Procédé suivant la revendication 10, dans lequel les cellules épithéliales neurales normales sont soumises à une sous-culture dans un milieu sans sérum, dépourvu d'héréguline.

12. Lignée de cellules précurseurs épithéliales normales isolée par le procédé de la revendication 1.

13. Lignée de cellules précurseurs épithéliales normales suivant la revendication 13, qui est une lignée de cellules de rat.

14. Lignée de cellules précurseurs épithéliales normales suivant la revendication 13, qui est déposée dans l'American Type Culture Collection et qui porte le numéro de dépôt CRL 11993.

15. Composition comprenant la lignée cellulaire de la revendication 12 et un véhicule pharmaceutiquement acceptable.

16. Composition comprenant la lignée cellulaire de la revendication 13 et un véhicule pharmaceutiquement acceptable.

17. Composition comprenant la lignée cellulaire de la revendication 14 et un véhicule pharmaceutiquement acceptable.
